# EUROPEAN PATENT APPLICATION

(11) **EP 1 269 974 A1**
(43) Date of publication of application: **02.01.2003**
(21) Application number: 01305286.5
(22) Date of filing: 18.06.2001
(51) Int. Cl.: A61K 7/11, A61K 7/06

(54) **Hair styling compositions**

(71) Applicant: UNILEVER PLC, London EC4P 4BQ (GB)
(72) Inventor: The designation of the inventor has not yet been filed
(74) Representative: Tansley, Sally Elizabeth

(57) **Abstract**

Hair styling compositions comprise from 0 to 35% by weight of an aerosol propellant and from 0.01% to 30% by weight wax particles in aqueous dispersion. The wax particles have an average particle size in the range of from 1 µm to 500 µm and a melting point in the range of from 30°C to 150°C. The compositions, which may be in the form of a foaming aerosol hair mousse, have good styling, softness and/or conditioning properties, whilst reducing the feeling of stickiness on the hands or the hair which is associated with corresponding compositions without the wax particles.

## Description

### Field of the Invention

This invention relates to hair styling compositions and to a method of styling hair using the compositions.

### Background of the Invention

The desire to have the hair retain a particular shape or style is widely held. The most common approach for accomplishing styling of hair is the application of a composition to dampened hair, after shampooing and/or conditioning, or to dry, styled hair. These compositions provide temporary styling benefits and can readily be removed by water or shampooing. To date, the materials employed in hair care compositions to provide styling benefits have generally been natural or synthetic resins and have been applied in the form of, for example, sprays, mousses, gels and lotions.

Style creation products such as hair styling mousses provide human hair with a temporary set which can be removed by water or by shampooing, and function by applying a thin film of a resin or gum onto the hair to adhere adjacent hairs together so that they retain the particular shape or configuration at the time of application.

Since hair styling compositions are intended to adhere hairs together, they can sometimes impart a sticky feeling, particularly on the hands if the user applies the composition to the hands before rubbing it into the hair. This mode of applying the composition to the hair is typically employed when a hair styling mousse is used. In order to reduce any undue stickiness in hair cosmetic preparations and glazing agents, US 6,066,316 proposes the use of a fine dispersion of wax in combination with a specific mixture of surfactants. The wax particles used in this document have a size of 30 nm and dispersions of this type are commonly termed microemulsions or microdispersions.

Cosmetic compositions containing wax particles having a size less than 500 nm are taught in US 5,306,488 and US 5,985,255. The compositions are claimed to impart volume to the hair and to thicken fine hair.

Wax-based compositions for use in lipsticks and lip-care products are described in WO 98/53793. The compositions are water-in-oil emulsions and, therefore, do not contain an aqueous dispersion of wax particles.

The use of wax in mascara products, together with a mixture of sugar esters, is described in US 5,866,111. There is no disclosure of the size of the wax particles in this document.

WO 00/33808 discloses hair styling compositions containing an organic phospholipid capable of forming bilayers in aqueous solution, a specific surfactant mixture and a water-insoluble ingredient which may be a wax. There is no mention in the document of the particle size of the wax and the teaching of the document is directed to the use of the organic phospholipid as a delivery agent for the water-insoluble component.

Wax particles containing a perfume are described in US 4,152,272. This document primarily relates to the delivery of perfume from fabric conditioners and the wax acts solely as a carrier for the perfume.

The present invention is based on the surprising finding that hair styling compositions comprising wax particles have improved properties if the average particle size of the particles is within a specific range and is larger than that disclosed in the styling compositions of the prior art, such as, for example US 6,066,316. The use of larger wax particles has the surprising advantage of providing improved conditioning properties, leading for example to improved softness and improved ease of styling (such as ease of comb/brushing) of hair treated with the compositions, compared to hair treated with compositions containing smaller wax particles. Moreover, these advantages are achieved without adverse effects on the beneficial properties of the compositions containing the smaller wax particles, such as reduced stickiness on hands and on wet hair.

### Summary of the Invention

According to a first aspect of the invention, there is provided a hair styling composition comprising from 0.001% to 10% by weight of a hair styling polymer, from 0 to 35% by weight of an aerosol propellant and from 0.01% to 30% by weight of wax particles in aqueous dispersion in the composition, wherein the wax particles have an average particle size in the range of from 1 µm to 500 µm and comprise a wax having a melting point in the range of from 30°C to 150°C.

In a second aspect, the invention provides a hair styling composition comprising from 0 to 35% by weight of an aerosol propellant and from 0.01% to 30% by weight wax particles in aqueous dispersion in the composition, wherein the wax particles have an average particle size in the range of from 1 µm to 500 µm and comprise at least 95% by weight of the particles of a wax having a melting point in the range of from 30°C to 150°C.

In a third aspect, the present invention provides a hair styling composition, which is in the form of a foaming aerosol hair mousse, comprising from 2 to 30% by weight of an aerosol propellant and from 0.01% to 30% by weight wax particles in aqueous dispersion in the composition, wherein the wax particles have an average particle size in the range of from 1 µm to 500 µm and comprise a wax having a melting point in the range of from 30°C to 150°C.

A fourth aspect of the invention is a method of styling hair which comprises applying to the hair a composition of the invention.

### Detailed Description of the Invention

### The wax particles

The invention is based on the surprising finding that the properties of hair styling compositions can be improved by incorporating in the compositions wax particles having a specified average particle size. The wax particles comprise at least one wax which is a hydrophobic material which is solid at room temperature (25°C) and is substantially insoluble in water (ie, it has a water solubility of less than 0.01g/l) at room temperature (25°C).

The wax particles are included in the compositions in an amount of from 0.01% to 30% by weight, preferably from 0.1% to 10% by weight, most preferably 0.1% to 2.5% by weight, based on the total weight of the composition. The wax particles are in aqueous dispersion in the composition, that is to say that the particles represent a distinct wax phase which is surrounded by a liquid phase comprising water(preferably, the liquid phase comprises greater than 50% water).

The wax particles have an average particle size in the range of from 1 µm to 500 µm, preferably the wax particles have an average size in the range of from 1 µm to 150 µm, more preferably from 5 µm to 150 µm, most preferably from 10 µm to 150 µm. Average particle size is based on the average particle size by volume [d (0.5)] and can be determined by conventional methods, such as by light scattering analysis using a Malvern Mastersizer apparatus (Malvern Instruments, Malvern, UK).

The wax has a melting point in the range of from 30°C to 150°C, preferably the wax has a melting point in the range of from 40°C to 100°C, for example from 45°C to 80°C. It has been found that waxes having melting points above about 45°C have improved hair conditioning properties, in certain compositions of the invention. Melting points refer to the melting point of the material which makes up the wax particles, including the effect of any further component or components in the particles in addition to the wax itself.

The particles preferably comprise the wax in an amount of greater than 96% by weight of the wax particles, however lower amounts of wax are also possible. Other components that may be included in the wax particles include, for example, solvents, plasticisers and hair benefit agents; typically these will have a greater solubility in the wax phase than in the aqueous phase.

The chemical identity of the wax is less important than the physical properties of the wax and suitable waxes include those from synthetic and natural sources. The waxes are typically non-polymeric and their major component may have a molecular weight in the range of from 100 to 2,000 Daltons, more preferably from 100 to 1,000 Daltons. Preferably, the wax is selected from naturally occurring waxes, synthetic hydrocarbon waxes, synthetic silicone waxes and mixtures thereof. Naturally occurring waxes may be obtained directly or indirectly from natural plant, animal or mineral sources. Suitable waxes from natural sources include those based on triglycerides, for example waxes obtained by the hydrogenation of vegetable oil, animal fats and oils and natural waxes from plants. Suitable synthetic waxes include silicone waxes and hydrocarbon waxes. Modified fatty acid (fatty acids include carboxylic acids containing from 12 to 24 carbon atoms) glycerol esters are also suitable for use in the invention. Waxes may contain substantially one chemical compound or a mixture of chemical compounds and can be used singly or as a mixture of two or more different waxes. Preferred waxes are those based on hydrogenated vegetable oil, candelilla wax (extracted from the candelilla plant) carnauba wax (extracted from the palm tree carnauba) and dimethicone polyol waxes. Candelilla wax is particularly preferred.

Examples of other waxes that are suitable for use in the invention include beeswax, cotton wax, bayberry wax, Chinese wax, spermaceti, montan wax, rice bran wax, lanolin, kapok wax, lanolin acetate or other lanolin esters and/or ethers, sugar cane wax, hexyl laurate, jojoba wax, shellac, paraffin wax, cholesterol, hydrogenated castor oil, petrolatum, cacao butter, coconut oil, palm oil, palm kernel oil, and the like, optionally hydrogenated where this is not already specified and where this is appropriate in order to increase the melting point of the wax such that it is within the range of from 30°C to 150°C.

The waxes may contain liquid fats and/or oils, provided that the wax remains substantially solid (which term includes self-supporting soft solids) at room temperature (25°C). Liquid fats and oils include triglyceride oils from plant sources, such as for example avocado oil, olive oil, corn oil, rape seed oil, sesame oil, wheat germ oil, castor oil, linseed oil, sunflower oil, cottonseed oil, soybean oil, peanut oil, tea tree oil, jojoba oil and the like. Other liquid oils include fatty acids (ie, acids having more than 10 carbon atoms), fatty alcohols (ie, alcohols having more than 10 carbon atoms) and fatty acid esters (ie, esters formed between C1-C10 alcohols and fatty acids), for example butyl myristate, cetyl palmitate, decyl oleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, propylene glycol isostearate, behenic acid, palmitic acid, stearic acid, behenyl alcohol, cetyl alcohol, isocetyl alcohol and mixtures thereof.

### Hair Styling Polymer

The compositions of the invention in its first aspect comprise from 0.001% to 10% by weight of a hair styling polymer. The compositions of the second and third aspects of the invention are compositions in which the presence of a hair styling polymer is not essential. However, the compositions of the second and third aspects of the invention preferably also comprise from 0.001% to 10% by weight of a hair styling polymer.

More preferred amounts of hair styling polymer in the compositions of the invention are from 0.1% to 5% by weight of the composition, even more preferably from 0.5% to 3% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.
The additional styling polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP Corporation esterified copolymers of methyl vinyl ether and maleic anhydride);
Luviset PUR® available from BASF.

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as additional styling polymers in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

In certain embodiments of the invention, the styling polymer is preferably a copolymer having a backbone comprising a polyether and, depending from the backbone, a plurality of poly (vinyl ester) groups. At least some of the ester groups are hydrolysed to the corresponding alcohol, preferably at least 50%, more preferably at least 75%, most preferably at least 95% of the groups are hydrolysed to the corresponding alcohol. The poly (vinyl ester) chains optionally contain other functional groups in and/or on the polymer chain, such as, for example, amide and/or keto groups. The copolymer has a polyether backbone which is obtainable by the polymerisation of one or more alkylene oxides. The polyether may comprise a single alkyleneoxy group or a mixture of two or more alkyleneoxy groups. The polyether may, for example, be based on ethylene oxide, propylene oxide, butylene oxide, other alkylene oxides, polyglycerol and mixtures thereof. Optionally, the backbone comprises linkages other than those based on polyether, such as, for example, amide or keto linkages. Preferably, the copolymer comprises a polyethyleneglycol backbone. The copolymer is preferably polyethyleneglycol-copolyvinylalcohol having polyvinylalcohol groups bound to the polyethyleneglycol i.e., subtantially all of the poly (vinyl ester) groups are preferably hydrolysed in the copolymers used in the compositions of the invention. The copolymer can be produced by methods which are well-known to those skilled in the art. For example, the copolymers are obtainable by graft polymerisation. In a method comprising graft polymerisation, poly (vinyl ester) groups are preferably grafted onto a polyether and are subsequently hydrolysed to convert at least some of the ester groups to the corresponding alcohol. For example, DE 1 077 430, the contents of which are incorporated herein by reference, describes a process for the preparation of graft polymers of vinyl esters on polyalkylene glycols. The preparation of graft copolymers of polyvinyl alcohol on polyalkylene glycols by hydrolysis of the vinyl esters is described in DE 1 094 457 and DE 1 081 229, both also incorporated herein by reference. The weight average molecular weight of the polyether is preferably from 1 to 100 kDa. Preferred copolymers for use in compositions of the invention have a molar ratio of polyether to total poly(vinyl ester) and polyvinylalcohol groups in the range of from about 95:5 to 5:95, more preferably about 30:70 to about 50:50. Typically, such copolymers have a molar ratio of polyether to total poly(vinyl ester) and polyvinylalcohol groups of about 40:60. The copolymer may be non-cross-linked or cross-linked and it is preferred that the copolymer is cross-linked. Suitable cross-linking agents are those compounds which can bind to two or more polyether, poly (vinyl ester) and/or poly (vinyl alcohol) chains and include, for example, pentaerythritol triallyl ether.

### Surfactant

The compositions of the invention may comprise a surfactant. The compositions preferably comprise from 0.01% to 5% by weight of a surfactant. The surfactants which are suitable for use in the compositions of the invention may be nonionic, cationic, anionic, zwitterionic or a mixture of such surfactants depending on the product form.

The hair styling compositions of the invention preferably comprise a non-ionic surfactant, in an amount of up to 5%, preferably from 0.01% to 1%, most preferably from 0.02% to 0.8% by weight based on total weight.

Examples of suitable non-ionic surfactants are condensation products of aliphatic (C₈-C₁₈) primary or secondary linear or branched chain alcohols or phenols with alkylene oxides, usually ethylene oxide and generally having at least 15, preferably at least 20, most preferably from 30 to 50 ethylene oxide groups. Other suitable non-ionics include esters of sorbitol, esters of sorbitan anhydrides, esters of propylene glycol, fatty acid esters of polyethylene glycol, fatty acid esters of polypropylene glycol, ethoxylated esters and polyoxyethylene fatty ether phosphates.

Of particular use are those non-ionic surfactants of general formula R(EO)ₓ H, where R represents a straight or branched chain alkyl group having an average carbon chain length of 12-18 carbon atoms and x ranges from 30 to 50. Specific examples include steareth-40, steareth-50, ceteareth-30, ceteareth-40, ceteareth-50 and mixtures thereof. Suitable commercially available examples of these materials include Unicol SA-40 (Universal Preserv-A-Chem), Empilan KM50 (Albright and Wilson), NONION PS-250 (Nippon Oils & Fats), Volpo CS50 (Croda Inc), and Incropol CS-50 (Croda Inc).

### Water

Compositions of the present invention will also include water, preferably distilled or de-ionised, as a solvent or carrier for the polymers and other components. Water will typically be present in amounts ranging from 30% to 98%, preferably from 50% to 90% by weight.

### Hair conditioning agents

Hair conditioning agents such as hydrocarbons, silicone fluids, and cationic materials may be included in the compositions of the invention. Hair conditioning agents may typically be present in compositions of the invention in amounts of from 0.001% to 10% by weight, preferably 0.1% to 1% by weight. Hair conditioning agents may be single compounds or mixtures of two or more compounds from the same class or different general classes.

Hair conditioning agents may be included in any of the compositions of the invention, regardless of whether they contain a hair styling polymer. In one embodiment of the invention, the compositions (such as aerosol mousse formulations, for example) comprise a hair conditioning agent and are substantially free of hair styling polymer.

Suitable hydrocarbons can be either straight or branched chain and can contain from about 10 to about 16, preferably from about 12 to about 16 carbon atoms. Examples of suitable hydrocarbons are decane, dodecane, tetradecane, tridecane, and mixtures thereof.

Examples of suitable silicone conditioning agents useful herein can include either cyclic or linear polydimethylsiloxanes, phenyl and alkyl phenyl silicones, and silicone copolyols. Cationic conditioning agents useful herein can include quaternary ammonium salts or the salts of fatty amines, such as cetyl ammonium chloride, for example.

Compositions according to the invention may, optionally, comprise from 0.1% to 10% by weight of a volatile silicone as the hair conditioning agent. Volatile silicones are well known in the art and are commercially available and include, for example linear and cyclic compounds. Volatile silicone oils are preferably linear or cyclic polydimethylsiloxanes containing from about three to about nine silicon atoms.

The compositions of the invention may optionally comprise a cross-linked silicone polymer.

The cross-linked silicone polymer is preferably a non-rigid emulsion-polymerised and may be present in compositions of the invention in an amount of up to 10% by weight based on the total weight of the composition, more preferably from 0.2% to 6% by weight, most preferably from 0.5 to 5% by weight.

Preferred silicone polymers for use in the invention are polydiorganosiloxanes, preferably derived from suitable combinations of R₃SiO_{0.5} units and R₂SiO units where each R independently represents an alkyl, alkenyl (e.g., vinyl), alkaryl, aralkyl, or aryl (e.g. phenyl) group. R is most preferably methyl.

The preferred silicone polymers of the invention are cross-linked polydimethyl siloxanes (which have the CTFA designation dimethicone), and cross-linked polydimethyl siloxanes having end groups such as hydroxyl (which have the CTFA designation dimethiconol). Good results have been obtained with cross-linked dimethiconol.

Cross-linking of the silicone polymer is typically introduced concurrently during emulsion polymerisation of the polymer through the inclusion of the required amount of trifunctional and tetrafunctional silane monomer units, for example, those of formula:

R Si (OH)₃

wherein R represents an alkyl, alkenyl (e.g. vinyl), alkaryl, aralkyl or aryl (e.g. phenyl) group, preferably methyl.

The degree of cross-linking of the silicone polymer can be measured as the percentage of branched monomer units in the silicone polymer and is from 0.05% to 10%, preferably being in the range 0.15% to 7%, e.g. from 0.2% to 2%. Increasing cross-linking is found to improve styling benefits but also to reduce conditioning performance somewhat, so compromise levels must be selected with properties optimised to suit consumer preferences in different cases. Good overall performance has been obtained with dimethiconol 0.3% cross-linked.

Suitable emulsion polymerised cross-linked silicone polymers are commercially available or can be readily made using conventional techniques well known to those skilled in the art.

Cross-linked silicone polymers are described in EP 818190, the contents of which are incorporated herein by reference.

### Product Form

Compositions of the present invention are formulated into hair styling compositions which may take a variety of forms, including, for example, mousses, gels, lotions, creams and tonics. These product forms are well known in the art.

The compositions of the first and second aspects of the invention are preferably foaming compositions. Foaming compositions are those compositions which are capable of forming a foam on dispensation from a suitable container, such as a pressurised aerosol container. More preferably, like the compositions of the third aspect of the invention, the compositions of the first and second are in the form of an aerosol hair mousse. Aerosol hair mousse compositions are emitted from the aerosol container as a foam which is then typically worked through the hair with fingers or a hair styling tool and either left on the hair or rinsed out.

Aerosol-form compositions of the invention will include an aerosol propellant which serves to expel the other materials from the container, and forms the mousse character in mousse compositions. The aerosol propellant included in styling compositions of the present invention can be any liquefiable gas conventionally used for aerosol containers. Examples of suitable propellants include dimethyl ether and hydrocarbon propellants such as propane, n-butane and iso-butane. The propellants may be used singly or admixed. Water insoluble propellants, especially hydrocarbons, are preferred because they form emulsion droplets on agitation and create suitable mousse foam densities.

The amount of the propellant used is governed by normal factors well known in the aerosol art. For mousses the level of propellant is generally up to 35%, preferably from 2% to 30%, most preferably from 3% to 15% by weight based on total weight of the composition. If a propellant such as dimethyl ether includes a vapour pressure suppressant (e.g. trichloroethane or dichloromethane), for weight percentage calculations, the amount of suppressant is included as part of the propellant.

Preferred propellants are selected from propane, n-butane, isobutane, dimethyl ether and mixtures thereof. Preferably, the propellant comprises dimethyl ether and at least one of propane, n-butane and isobutane.

The method of preparing aerosol hair styling mousse compositions according to the invention follows conventional aerosol filling procedures. The composition ingredients (not including the propellant) are charged into a suitable pressurisable container which is sealed and then charged with the propellant according to conventional techniques.

Compositions of the invention may also take a non-foaming product form, such as a hair styling cream or gel. Such a cream or gel will include a structurant or thickener, typically at a level of from 0.1% to 10%, preferably 0.5% to 3% by weight based on total weight.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01% to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with allylsucrose or allylpentaerythritol as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. It is also possible to use inorganic thickeners such as bentonite or laponite clays.

The hair styling compositions of the invention can contain a variety of non-essential, optional components suitable for rendering the compositions more aesthetically acceptable or to aid use, including discharge from the container, of the product. Such conventional optional ingredients are well known to those skilled in the art, e.g. preservatives such as benzyl alcohol, methyl paraben, propyl paraben and imidazolidinyl urea, fatty alcohols such as cetearyl alcohol, cetyl alcohol and stearyl alcohol, pH adjusting agents such as citric acid, succinic acid, sodium hydroxide and triethanolamine, colouring agents such as any of the FD&C or D&C dyes, perfume oils, chelating agents such as ethylenediamine tetraacetic acid, and polymer plasticising agents such as glycerin and propylene glycol.

The following non-limiting Examples further illustrate the preferred embodiments of the invention. All percentages referred to in the examples and throughout the specification are by weight of active ingredient based on total weight of the composition unless otherwise indicated.

### EXAMPLES

### Examples 1 to 17

Hair styling mousse compositions according to the invention were formulated according to the following general method.

### 1. Emulsification of Wax (for compositions according to the invention)

The wax (60 g) was melted along with the emulsifier(s) [Brij-72 (4 g), Myri-52S (6 g) and cetyl alcohol (8 g)] or [CTAC (16 g)]. The melt was slowly poured into hot water (∼324 g; at 20°C above the melting point of the wax) whilst being stirred at 500-2000 rpm. The heat was removed and the emulsion was left to cool to room temperature with stirring. As the emulsion cools through the melting point temperature the mixture thickens to give a cream. The emulsions manufactured by this process were ∼15% active by weight of wax.

### 2. Preparation of Microemulsion Carnauba Wax (for Control 2 example)

Carnauba wax (60 g) and palmitic acid (12 g) were melted together using a water bath and stirred using an overhead mechanical blade (20-100 rpm). Morpholine (9 g) was added carefully to the melt. Aliquots (∼ 3 g) of boiling water were slowly added to the melt, as more water was added, the molten wax darkened and became more viscous. After adding a total of 294 g of water the clear brown melt became less viscous and the wax microemulsion was left to cool to room temperature with slow stirring. The cool liquid was filtered through glass wool to remove any solids. The microemulsion manufactured by this process was ∼16% active by weight of wax.

### 3. Compositions Comprising A Polymer Base

A PEG-vinyl alcohol hair styling polymer was diluted with the total amount of water and heated to 80 °C to disperse the polymer. The clear solution was cooled to room temperature and surfactant was added into the mix. The wax emulsion was added to the cold solution with stirring (at this point if needed silicone emulsion was added). Preservatives and perfume were stirred in, the can was filled, crimped and gassed with propellant.

### 4. Non-polymer Base Compositions

Wax emulsion was diluted with the total amount of water and the surfactant was stirred in at room temperature. A cyclic silicone emulsion was stirred in along with the preservatives and perfume. The can was filled, crimped and gassed with propellant.

### 5. Foam Properties

Both type of mousse (ie, with and without a polymer base) gave stable, creamy foams 24 hr after gassing. Foam weights (densities) were measured by the weight of a known volume (50ml) at room temperature (five replications were made).

The materials used in the examples were obtained from the following sources:

| **Material** | **Source** |
|---|---|
| Polycerin hydro wax 30407 P | Tromm |
| Polycerin hydro wax 30618 | Tromm |
| Polycerin hydro wax 30620 | Tromm |
| Carnauba Wax (emulsion) | Aldrich |
| Carnauba Wax (microemulsion) | Aldrich |
| Candelilla Wax | Aldrich |
| DC2501 silicone wax | Dow corning |
| PEG/VOH [30%] hair styling polymer | BASF |
| Volpo CS50 | Croda |
| (CTAC) cetylammonuim chloride | Akzo Nobel |
| Brij 72 | Uniqema |
| Myrj 52S | Uniqema |
| Cetyl alcohol | Cognis |
| Emulsified cyclic silicone | Dow corning |
| DBDCB - 1,2-dibromo-2,4-dicyanobutane | Nalco |
| DMDM Hydantoin - 5,5 (1,3 dihydroxymethyl) dimethyl hydantoin | Sharon Laboratories |
| Perfume Vigorflor UN 131.786/F | Firmenich |
| Propellant A50/DME (60:40) | Du Pont |
| Deionised water | Local supply |

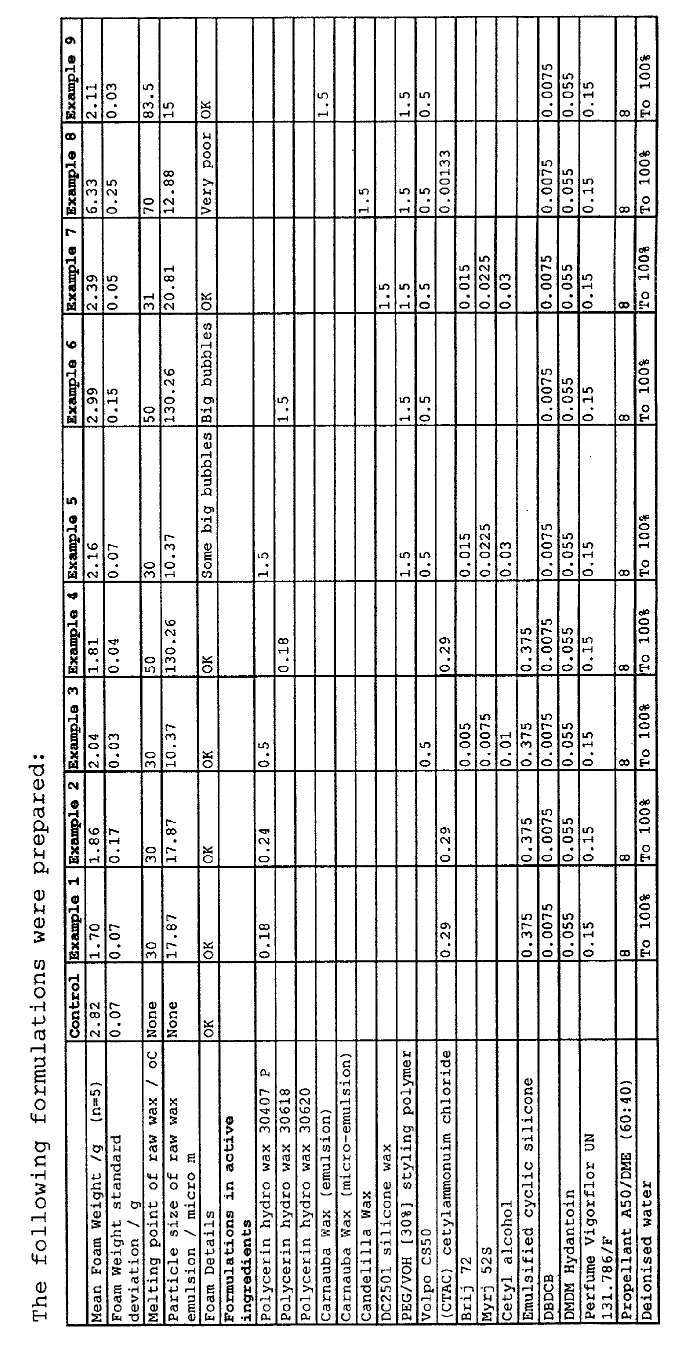

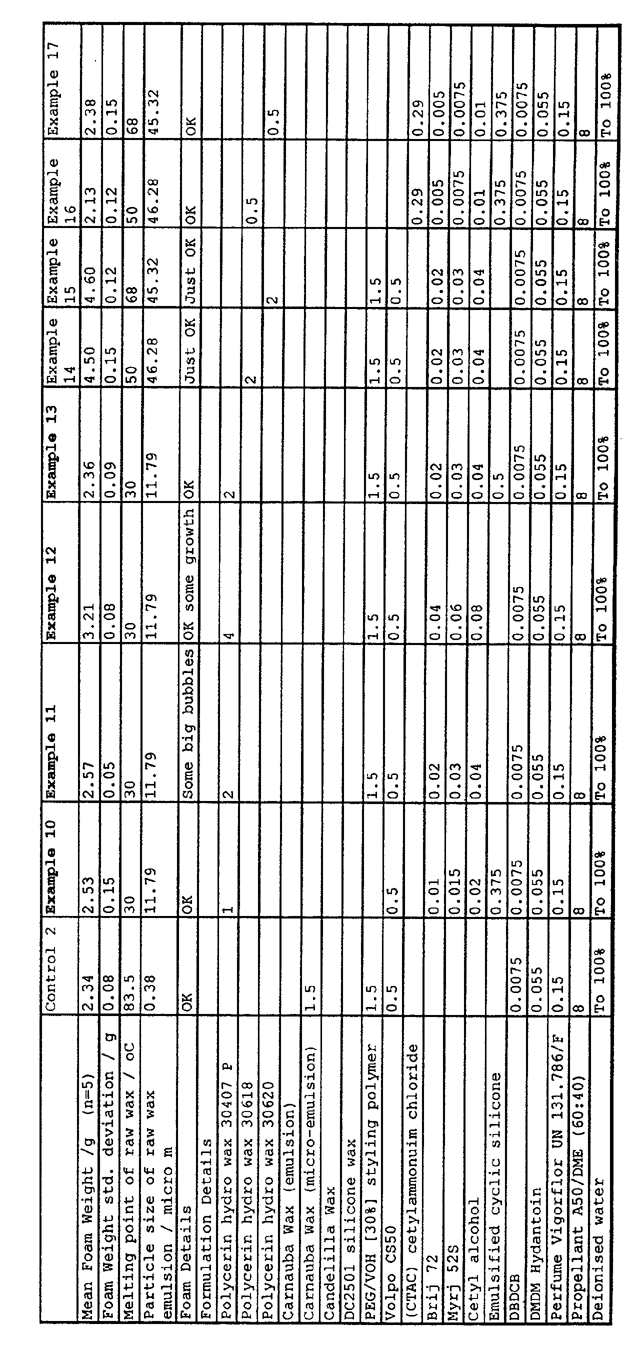

### Example 18

### Effect of emulsion particle size

A comparative test was carried out to determine the effect of wax emulsion particle size on the properties of the compositions of the invention.

The following compositions were compared to the Control:

| | |
|---|---|
| Example 9 | 1.5% Carnauba wax emulsion + 1.5% Polymer |
| Control 2 | 1.5% Carnauba wax microemulsion + 1.5% Polymer |

Testing was conducted by application of the formulations to half of the total head of hair by stylists. After stylist application, the panelist styled her own hair following her normal routine using her own styling tools. Each panelist then completed a questionnaire on the performance of each product. The results are given in the following table.

| **Means summary table for Example 18** | | | |
|---|---|---|---|
| | Control | Example 9 | Control 2 |
| N= | 40 | 34 | 34 |

| **Application** | | | |
|---|---|---|---|
| 1.) How sticky does the product leave your hands? | 5.1 | 3.4 | 3.7 |

| **Wet Hair** | | | |
|---|---|---|---|
| 2) How sticky does your hair feel? | 4.7 | 2.9 | 2.7 |
| 3) How slippery does your hair feel? | 5.9 | 4.5 | 3.8 |
| 4) How easy is it to comb/brush your hair? | 6.8 | 7.9 | 7.9 |
| 5) How Easy is it to Style your hair? | 6.6 | 7.2 | 6.4 |
| 6) How much grip/pull did you notice during styling? | 4.3 | 3.8 | 3.8 |

| **Dry Stage:** | | | |
|---|---|---|---|
| 7) How Easy is it to comb/brush your hair? | 6.8 | 7.5 | 7.1 |
| 8) How fast did your hair dry? | 5.5 | 5.8 | 5.9 |
| 9) How stiff is your hair? | 3.9 | 3.7 | 4.5 |
| 10.) How Conditioned does your hair feel? | 5.7 | 6.0 | 5.0 |
| 11.) How Dry Feeling is your hair? | 3.7 | 3.9 | 4.5 |
| 12.) How Soft is your hair? | 5.9 | 6.4 | 4.9 |
| 13.) How much clumping do you have? | 2.6 | 2.8 | 3.5 |
| 14.) How much flaking is on your hair? | 1.7 | 1.5 | 1.5 |
| 15.) How Flat / Limp is your hair? | 3.9 | 3.8 | 3.6 |
| 16.) How much Fullness do you have? | 5.6 | 5.5 | 5.6 |
| 17) How much Body does your hair have? | 5.8 | 5.5 | 5.6 |
| 18.) Amount of frizzies? | 2.4 | 2.5 | 2.2 |
| 19) How much static does your hair have? | 2.3 | 2.2 | 2.0 |
| 20) How much hold does the product have? | 6.0 | 5.8 | 5.4 |
| 21) How easy is it to curl your hair? | 6.5 | 6.1 | 5.9 |
| 22) Overall Liking | 6.0 | 6.1 | 5.5 |

Compared to the Control, both Example 9 and Control 2 were rated significantly less 'sticky on hands and in wet hair', less 'slippery', and 'easier to comb/brush wet hair'. Control 2 was rated significantly less 'soft' and had more 'clumping' versus the control

Example 9 with the larger emulsion particle size had significant wins in 'conditioning feeling' and 'softness' over Control 2. A directional win in 'ease of styling' was also recorded, however, all the other attributes scored the same.

The particle size does not appear to have a major impact on styling performance. However, surprisingly, the microemulsion sized wax particles seem to negatively contribute to the feel performance by decreasing 'softness' and 'conditioning feeling' and by increasing 'clumping'.

### Example 19

The following is an example of how another composition of the invention may be formulated.

In this example, wax emulsion refers to the emulsion containing Polycerin hydro wax 30407 P in a particle size of 17.87 µm and the amount is based on the amount of wax. The materials used in the examples include the following:

| **Material** | **Supplier** | **Function** |
|---|---|---|
| Sepicide LD™ | Seppic | Preservative |
| Cremophor RH410™ | BASF | Stabiliser |
| Carbopol 980™ | BF Goodrich | Structurant |
| Jaguar HP-105™ | Rhodia | Conditioning |
| Luviset PUR* | BASF | Adhesive |

A styling gel is formulated as follows:

| **Material** | **% w/w** |
|---|---|
| Luviset PUR | 3.8 |
| Carbopol 980 | 0.4 |
| Water | to 100% |
| Sepicide LD | 0.4 |
| Sodium hydroxide (8% 2M) | 0.1 |
| Cremaphor RH410 | 0.4 |
| Wax emulsion | 10 |
| Jaguar HP-105 | 0.2 |
| Perfume | 0.15 |

## Claims

1. Hair styling composition comprising from 0.001% to 10% by weight of a hair styling polymer, from 0 to 35% by weight of an aerosol propellant and from 0.01% to 30% by weight of wax particles in aqueous dispersion in the composition, wherein the wax particles have an average particle size in the range of from 1 µm to 500 µm and comprise a wax having a melting point in the range of from 30°C to 150°C.

2. Hair styling composition comprising from 0 to 35% by weight of an aerosol propellant and from 0.01% to 30% by weight wax particles in aqueous dispersion in the composition, wherein the wax particles have an average particle size in the range of from 1 µm to 500 µm and comprise at least 95% by weight of the particles of a wax having a melting point in the range of from 30°C to 150°C.

3. Composition as claimed in claim 2, which comprises from 0.001% to 10% by weight of a hair styling polymer.

4. Composition as claimed in any one of claims 1 to 3, wherein the hair styling polymer is a copolymer having a backbone comprising a polyether and, depending from said backbone, a plurality of poly(vinyl ester) groups, wherein at least some of the ester groups are hydrolysed to the corresponding alcohol.

5. Composition as claimed in any one of claims 1 to 4, which is a foaming composition.

6. Composition as claimed in claim 5 which is an aerosol hair mousse.

7. Hair styling composition, which is in the form of a foaming aerosol hair mousse, comprising from 2 to 30% by weight of an aerosol propellant and from 0.01% to 30% by weight wax particles in aqueous dispersion in the composition, wherein the wax particles have an average particle size in the range of from 1 µm to 500 µm and comprise a wax having a melting point in the range of from 30°C to 150°C.

8. Composition as claimed in any one of claims 1 to 7, which further comprises from 0.01% to 5% by weight of a surfactant.

9. Composition as claimed in any one of claims 1 to 8, which further comprises from 0.001% to 10% by weight of a hair conditioning agent.

10. Composition as claimed in any one of claims 1 to 9, wherein the wax has a melting point in the range of from 40°C to 100°C.

11. Composition as claimed in any one of claims 1 to 10, wherein the wax particles have an average size in the range of from 1 µm to 150 µm.

12. Composition as claimed in any one of claims 1 to 11, which comprises from 0.1% to 2.5% by weight wax particles.

13. Composition as claimed in any one of claims 1 to 12, wherein the wax is selected from naturally occurring waxes, synthetic hydrocarbon waxes, synthetic silicone waxes and mixtures thereof.

14. Composition as claimed in any one of claims 1 to 13, wherein the propellant is selected from propane, n-butane, isobutane, dimethyl ether and mixtures thereof.

15. Composition as claimed in any one of claims 1 to 14, further comprising water in an amount of from 50% to 90% by weight.

16. A method of styling hair which comprises applying to the hair a composition of any one of claims 1 to 15.
